# EUROPÄISCHE PATENTSCHRIFT

(11) **EP 3 883 641 B1**
(45) Veröffentlichungstag und Bekanntmachung des Hinweises auf die Patenterteilung: **12.06.2024**
(21) Anmeldenummer: 19805197.1
(22) Anmeldetag: 12.11.2019
(51) Int. Cl.: A61N 1/05, A61B 5/053, A61B 5/15, A61M 19/00, A61N 1/36, C23C 14/00

(54) **MULTIPOLARE KANÜLE**
MULTIPOLAR CANNULA
CANULE MULTIPOLAIRE

(30) Priorität: 23.11.2018 DE 102018129541
(43) Veröffentlichungstag der Anmeldung: 29.09.2021
(73) Patentinhaber: Pajunk GmbH Medizintechnologie, 78187 Geisingen (DE)
(72) Erfinder: PAJUNK-SCHELLING, Simone, 78187 Geisingen (DE); HAUGER, Martin, 78166 Donaueschingen (DE)
(74) Vertreter: Westphal, Mussgnug & Partner Patentanwälte mbB
(86) Internationale Anmeldenummer: PCT/EP2019/081060
(87) Internationale Veröffentlichungsnummer: WO 2020/104259

(56) Entgegenhaltungen:
- WO-A1-97/15347
- DE-A1- 2 652 050
- DE-A1-102007 009 425
- HERNANDEZ ET AL: "MEASUREMENT OF BIO-IMPEDANCE WITH A SMART NEEDLE TO CONFIRM PERCUTANEOUS KIDNEY ACCESS", JOURNAL OF UROLOGY, LIPPINCOTT WILLIAMS & WILKINS, BALTIMORE, MD, US, Bd. 166, Nr. 4, 1. Oktober 2001 (2001-10-01), Seiten 1520-1523, XP005542762, ISSN: 0022-5347, DOI: 10.1016/S0022-5347(05)65823-5
- DENNIS TREBBELS ET AL: "Real-time cannula navigation in biological tissue with high temporal and spatial resolution based on impedance spectroscopy", 2010 ANNUAL INTERNATIONAL CONFERENCE OF THE IEEE ENGINEERING IN MEDICINE AND BIOLOGY SOCIETY : (EMBC 2010) ; BUENOS AIRES, ARGENTINA, 31 AUGUST - 4 SEPTEMBER 2010, IEEE, PISCATAWAY, NJ, USA, 31. August 2010 (2010-08-31), Seiten 1886-1889, XP032108845, DOI: 10.1109/IEMBS.2010.5627107 ISBN: 978-1-4244-4123-5

## Beschreibung

Die Erfindung betrifft eine multipolare Kanüle.

Bekannt sind multipolare Kanülen, beispielsweise bipolare Kanülen mit einer ersten Elektrode und einer zweiten Elektrode, welche gegeneinander elektrisch isoliert ausgebildet sind. Ein bekannter Aufbau besteht dabei darin, auf einen Kanülenrohrkörper eine elektrisch isolierende Schicht in Form eines Rohres aus isolierendem Kunststoff aufzubringen und einen weiteren elektrisch leitenden Rohrkörper auf das elektrisch isolierende Rohr aufzuschieben.

Als Stand der Technik werden die Veröffentlichung HERNANDEZ ET AL, "MEASUREMENT OF BIO-IMPEDANCE WITH A SMART NEEDLE TO CON-FIRM PERCUTANEOUS KIDNEY ACCESS", JOURNAL OF UROLOGY, LIP-PINCOTT WILLIAMS & WILKINS, BALTIMORE, MD, US, (20011001), vol. 166, no. 4, doi:10.1016/50022-5347(05)65823-5, ISSN 0022-5347, pages 1520 - 1523, XP005542762, die Veröffentlichung DENNIS TREBBELS ET AL, "Real-time cannula navigation in biological tissue with high temporal and spatial resolution based on impedance spectroscopy", 2010 ANNUAL INTERNATIONAL CONFER-ENCE OF THE IEEE ENGINEERING IN MEDICINE AND BIOLOGY SOCIETY : (EMBC 2010) ; BUENOS AIRES, ARGENTINA, 31 AUGUST - 4 SEPTEMBER 2010, IEEE, PISCATAWAY, NJ, USA, (20100831), doi:10.1109/IEMBS.2010.5627107, ISBN 978-1-4244-4123-5, pages 1886 - 1889, XP032108845 sowie die DE 10 2007 009 425 A1 genannt.

Die Aufgabe der Erfindung besteht daher darin, eine multipolare Kanüle weiterzubilden.

Die Aufgabe der Erfindung wird gelöst durch eine multipolare Kanüle mit den Merkmalen des Patentanspruchs 1.

Vorteilhafte Ausgestaltungen und Weiterbildungen der Erfindung sind in den abhängigen Ansprüchen angegeben.

Die erfindungsgemäße multipolare Kanüle weist ein Kanülenrohr mit einem distalen und einem proximalen Ende und eine erste Elektrode und wenigstens eine zweite Elektrode auf, wobei das Kanülenrohr einen Kanülenrohrkörper und eine die erste und die zweite Elektrode gegeneinander elektrisch isolierende Schicht aufweist, wobei das distale Ende des Kanülenrohrs eine distale Spitze aufweist, wobei die erste Elektrode durch den Kanülenrohrkörper gebildet ist und wobei die erste Elektrode und die zweite Elektrode mit einer Bioimpedanzmesseinheit verbindbar sind.

Durch eine derartige Ausgestaltung wird eine kompakt aufgebaute multipolare Kanüle bereitgestellt, mit welcher Bioimpedanzmessungen durchführbar sind. Für eine Bioimpedanzmessung wird der elektrische Widerstand zwischen den freien Enden der ersten und zweiten Elektrode ermittelt.

Erfindungsgemäß ist an dem proximalen Ende des Kanülenrohrs ein Ansatz angeordnet ist, welcher einen elektrisch kontaktierenden Anschluss für die Elektroden aufweist. Dadurch kann auf einfache Art und Weise eine elektrische Kontaktierung der Elektroden erreicht werden, insbesondere, wenn die Elektroden über die gesamte Länge des Kanülenrohrs vom distalen Ende bis zum dem elektrisch kontaktierenden Anschluss verlaufen.

Gemäß der Erfindung sind die erste Elektrode und die zweite Elektrode mittels eines Schalters wahlweise mit einer Stromversorgung oder mit der Bioimpedanzmesseinheit verbindbar. Werden die beiden Elektroden mit der Bioimpedanzmesseinheit verbunden, kann bestimmt werden, in welcher Art von Gewebe die Spitze der multipolaren Kanüle derzeit angeordnet ist. Andererseits können die beiden Elektroden im Rahmen der multipolaren Kanüle durch entsprechende Beaufschlagung zur Stimulation verwendet werden.

Vorzugsweise weist die multipolare Kanüle eine Auswerteeinheit auf oder ist mit einer Auswerteeinheit verbunden, die ausgebildet ist, die von den Elektroden kommenden elektrischen Signale auszuwerten und ein Anzeigesignal, beispielsweise in Form eines akustischen oder optischen Anzeigesignals, zu erzeugen. Das Anzeigesignal kann derart ausgebildet sein, dass ein Benutzer erkennen kann, in welcher Art von Gewebe die Spitze der multipolaren Kanüle derzeit angeordnet ist.

Vorzugsweise sind die elektrisch isolierende Schicht und wenigstens die zweite Elektrode in einem Dünnschichtverfahren auf den Kanülenrohrkörper aufgebracht. Die elektrisch isolierende Schicht ist dadurch insbesonders als elektrisch isolierende Beschichtung ausgebildet. Dadurch werden signifikant geringere Querschnitte der Kanüle im Vergleich zu einer herkömmlichen Kanüle in Form eines Doppelrohres ermöglicht.

Gemäß einer besonders bevorzugten Ausführungsform weist die elektrisch isolierende Schicht eine Dicke von wenigen Mikrometern, vorzugsweise eine Dicke von weniger als 1 Mikrometer auf. Dadurch können die äußeren Dimensionen im Querschnitt der multipolaren Kanüle signifikant verringert werden.

Vorzugsweise weist die zweite Elektrode eine Dicke von wenigen Mikrometern, vorzugsweise eine Dicke von weniger als 1 Mikrometer, auf. Dadurch kann der Durchmesser der multipolaren Kanüle deutlich verringert werden.

Gemäß einer bevorzugten Ausführungsform ist die elektrisch isolierende Schicht aus Parylen. Parylene eignen sich für die Beschichtung auf unterschiedlichsten Substratmaterialien und zur Beschichtung von unterschiedlichsten geometrischen Körpern, so dass sie für die Beschichtung von Kanülenrohrkörpern besoders geeignet sind.

Vorzugsweise bedeckt die elektrisch isolierende Schicht einen distalen Abschnitt des Kanülenrohrkörpers abgesehen von der distalen Spitze oder im Wesentlichen vollständig. Dadurch kann eine gute Isolierung zwischen dem Kanülenrohrkörper und in oder auf der isolierenden Schicht angebrachten zweiten Elektroden ermöglicht werden.

Vorteilhafterweise ist die zweite Elektrode im Dünnschichtverfahren auf die elektrisch isolierende Schicht aufgebracht, wodurch eine geringe Schichtdicke der zweiten Elektrode realisiert werden kann.

Besonders bevorzugt ist die zweite Elektrode aus Aluminium, da Aluminium eine gute elektrische Leitfähigkeit aufweist, und zudem auf verschiedenen Materialien wie beispielsweise Parallenen, gut haftet.

Vorteilhafterweise ist die zweite Elektrode beabstandet zum distalen Ende der elektrisch isolierenden Schicht angeordnet und bedeckt insbesondere die elektrisch isolierende Schicht abgesehen von einem distalen ringförmig umlaufenden Abschnitt. Durch die Beabstandung zum distalen Ende der elektrisch isolierenden Schicht kann eine gute elektrische Isolierung zwischen der zweiten Elektrode und dem Kanülenrohrkörper ermöglicht werden. Bedeckt die zweite Elektrode die elektrisch isolierende Schicht abgesehen von einem distalen ringförmig umlaufenden Abschnitt, kann eine großflächige zweite Elektrode mit guten elektrisch leitenden Eigenschaften bereitgestellt werden.

Vorteilhafterweise ist auf der zweiten Elektrode zumindest abschnittsweise eine zweite elektrisch isolierende Schicht angeordnet.

Vorteilhafterweise ist die zweite elektrisch isolierende Schicht aus Parylenen oder Weißlack. Insbesondere für den Fall, dass die zweite Elektrode aus Aluminium gefertigt ist, bietet sich als zweite elektrisch isolierende Schicht ein Weißlack an, um die Leitfähigkeit der Aluminiumschicht möglichst wenig zu beeinträchtigen.

Vorzugsweise bedeckt die zweite elektrisch isolierende Schicht die zweite Elektrode abgesehen von wenigstens einem distal angeordneten aktiven Abschnitt, um eine sichere Handhabung der Kanüle durch einen Benutzer zu ermöglichen. Es ist möglich, dass jede der zweiten Elektroden mehr als einen aktiven Abschnitt aufweist, wodurch komplexe Geometrien an Elektrodenstrukturen ermöglicht werden können.

Eine besonders bevorzugte Ausführungsform der Erfindung sieht vor, dass die zweite Elektrode in der elektrisch isolierenden Schicht angeordnet ist. Eine derartige Anordnung kann dadurch erreicht werden, dass die zweite Elektrode und die elektrisch isolierende Schicht gemeinsam auf den Kanülenrohrkörper aufgebracht werden. Dies ermöglicht eine Einbettung des zweiten Elektrode oder auch mehrerer zweiter Elektroden in die elektrisch isolierende Schicht.

Die Erfindung wird anhand der nachfolgenden Figuren ausführlich erläutert. Es zeigen:
- Figur 1:: eine schematische perspektivische Darstellung eines distalen Endes eines ersten Ausführungsbeispiels einer erfindungsgemäßen multipolaren Kanüle und
- Figur 2:: eine schematische perspektivische Darstellung eines distalen Endes eines zweiten Ausführungsbeispiels einer erfindungsgemäßen multipolaren Kanüle.

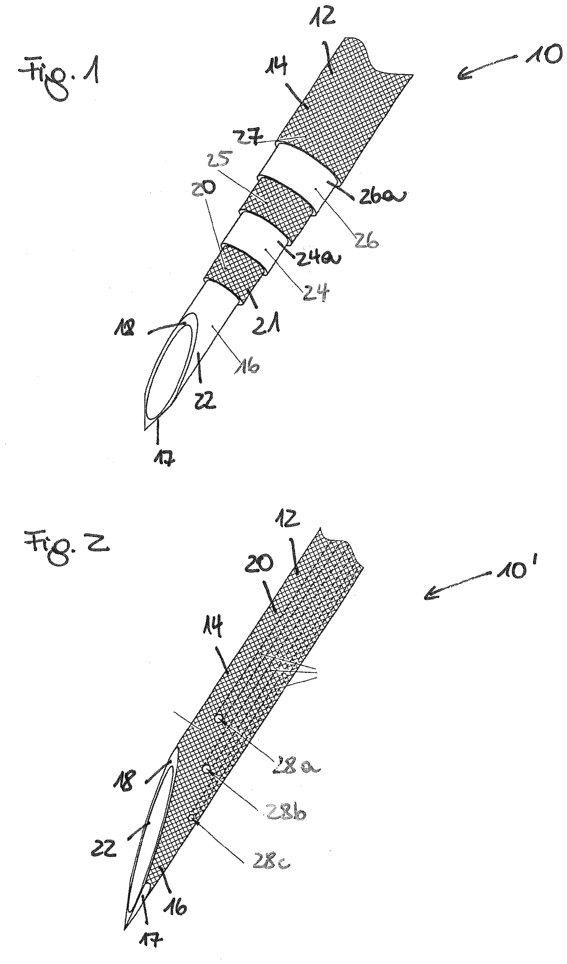

Figur 1 zeigt ein erstes Ausführungsbeispiel einer multipolaren Kanüle 10 mit einem Kanülenrohr 12 mit einem distalen Ende 14 und einem nicht dargestellten proximalen Ende. Das Kanülenrohr 12 weist einen Kanülenrohrkörper 18 und eine elektrisch isolierende Schicht 20 auf. Der Kanülenrohrkörper 18 bildet eine erste Elektrode 22. Daher ist der Kanülenrohrkörper 18 aus einem elektrisch leitenden Material gefertigt und beispielsweise als Stahlrohr ausgebildet.

Der Kanülenrohrkörper 18 weist am distalen Ende 14 eine distale Spitze 16 auf, welche beispielsweise dadurch gebildet sein kann, dass das distale Ende 14 in einen Winkel gegen die Längsachse des Kanülenrohrs 12 abgeschrägt verläuft, beispielsweise in einem Winkel von etwa 45°. Zusätzlich kann das distale Ende der distalen Spitze 16 einen Facettenschliff 17 aufweisen, um die Schärfe der distalen Spitze 16 zu steigern.

Die elektrisch isolierende Schicht 20 kann als elektrisch isolierende Beschichtung in einem Dünnschichtverfahren auf den Kanülenrohrkörper 18 aufgebracht und überdeckt den Kanülenrohrkörper 18 insbesondere umlaufend, wobei die distale Spitze 16 frei bleiben kann. Die elektrisch isolierende Schicht 20 kann bis zum proximalen Ende des Kanülenrohrkörpers 18 ausgebildet sein.

Auf der elektrisch isolierenden Schicht 20 ist eine zweite Elektrode 22 angeordnet, welche beispielsweise umlaufend um die elektrisch isolierende Schicht 20 derart angeordnet ist, dass das distale Ende der zweiten Elektrode 22 zum distalen Ende der elektrisch isolierenden Schicht 20 beabstandet ist und insbesondere ein ringförmig umlaufender Abschnitt 21 der elektrisch isolierenden Schicht 20 frei bleibt. Durch den umlaufenden Abschnitt 21 ist eine ausreichende elektrische Isolierung zwischen der ersten Elektrode 22 und der zweiten Elektrode 24 auch an den frei bleibenden aktiven Flächen gewährleistet. Die zweite Elektrode 22 kann sich dabei bis zum proximalen Ende des Kanülenrohrs 18 erstrecken.

Auf der zweiten Elektrode 24 ist eine zweite elektrisch isolierende Schicht 25 angeordnet, insbesondere derart, dass die zweite elektrisch isolierende Schicht 25 die zweite Elektrode 24 abgesehen von wenigstens einem distal angeordneten aktiven Abschnitt 24a bedeckt. Der aktive Abschnitt 24a kann beispielsweise ringförmig umlaufend ausgebildet sein oder nahezu jede beliebige geometrische Form annehmen, insbesondere als kreisförmige, elliptische oder rechteckige Fläche ausgebildet sein.

Sollen weitere Pole für eine wie zuvor beschriebene multipolare Kanüle 10 bereitgestellt werden, kann, wie in dem dargestellten Ausführungsbeispiel in Figur 1 ersichtlich, auf die zweite elektrisch isolierende Schicht 25 eine dritte Elektrode 26, vorzugsweise ebenfalls im Dünnschichtverfahren, aufgebracht werden, welche wiederum ebenfalls mit einer dritten elektrisch isolierenden Schicht 27 abgesehen von wenigstens einem distal angeordneten aktiven Abschnitt 26a abgedeckt ist. Auf die gleiche Art und Weise kann die Kanüle um weitere Pole ergänzt werden.

Wenigstens die erste Elektrode 22 und die zweite Elektrode 24 sind mit einer Bioimpedanzmesseinheit verbindbar.

Am proximalen Ende der multipolaren Kanüle 10 sind die Elektroden 22, 24, 26 elektrisch leitend kontaktiert, so dass über die Elektroden 22, 24, 26 eine elektrische Stimulation bei Einführen der multipolare Kanüle 10 in den Körper eines Patienten möglich ist. Dazu ist ein Ansatz am proximalen Ende des Kanülenrohrs angeordnet, welcher einen elektrisch kontaktierenden Anschluss für die Elektroden 22, 24, 26 aufweist.

Die erste Elektrode 22 und die zweite Elektrode 24 sind mittels eines nicht dargestellten Schalters mit einer Stromversorgung oder mit der Bioimpedanzmesseinheit verbindbar.

Die multipolare Kanüle kann eine Auswerteeinheit aufweisen oder mit einer Auswerteeinheit verbunden sein, die ausgebildet ist, die von den Elektroden kommenden elektrischen Signale auszuwerten und ein Anzeigesignal, beispielsweise in Form eines akustischen oder optischen Anzeigesignals, zu erzeugen. Figur 2 zeigt ein weiteres Ausführungsbeispiel einer multipolaren Kanüle 10', welche wie die multipolare Kanüle 10 gemäß dem ersten Ausführungsbeispiel das Kanülenrohr 12 mit einem distalen Ende 14 und einem nicht dargestellten proximalen Ende aufweist, welches ein Kanülenrohrkörper 18 und eine elektrisch isolierende Schicht 20 aufweist. Der Kanülenrohrkörper 18 stellt wiederum die erste Elektrode 22 dar.

Die multipolare Kanüle 10' gemäß dem zweiten Ausführungsbeispiel unterscheidet sich dadurch von dem ersten Ausführungsbeispiel, dass in der elektrisch isolierenden Schicht 20 wenigstens eine, im vorliegenden Ausführungsbeispiel drei zweite Elektroden 28a, 28b, 28c, eingebettet angeordnet sind. Die Elektroden 28a, 28b, 28c sind als Leiterbahnen in der elektrisch isolierenden Schicht 20 ausgebildet und verlaufen vom distalen Bereich des Kanülenrohrs 12 bis zum proximalen Ende. Sie können bis an die distale Spitze 16 des Kanülenrohrs 12 heranreichen. Die aktiven Bereiche der Elektroden 18a, 28b, 28c können durch Entfernen der elektrisch isolierenden Schicht 20 über den distalen Enden der Elektroden 28a, 28b, 28c freigelegt werden. In dem Ausführungsbeispiel sind die Elektroden 28a, 28b, 28c als im Wesentlichen runde zueinander parallel verlaufende Leiterbahnen ausgebildet. Es ist jedoch ersichtlich, dass die Elektroden vielfältige geometrische Ausgestaltungen annehmen können.

Ein weiterer Unterschied des zweiten Ausführungsbeispieles der multipolaren Kanüle 10` gegenüber dem ersten Ausführungsbeispiel besteht darin, dass die elektrisch isolierende Schicht 20 den gesamten Kanülenrohrkörper 18 bis über die distale Spitze 16 abdeckt und lediglich die Stirnfläche des Kanülenrohrkörpers 18 sowie gegebenenfalls vorhandene Facettenschliffflächen 17 frei lässt.

### Bezugszeichenliste

- 10: Multipolare Kanüle
- 10': Multipolare Kanüle
- 12: Kanülenrohr
- 14: distales Ende
- 16: distale Spitze
- 17: Facettenschliff
- 18: Kanülenrohrkörper
- 20: elektrisch isolierende Schicht
- 21: Abschnitt
- 22: erste Elektrode
- 24: zweite Elektrode
- 24a: aktiver Abschnitt
- 25: zweite elektrisch isolierende Schicht
- 26: dritte Elektrode
- 26a: aktiver Abschnitt
- 27: dritte elektrisch isolierende Schicht
- 28a: Elektrode
- 28b: Elektrode
- 28c: Elektrode

## Patentansprüche

1. Multipolare Kanüle (10, 10') mit einem Kanülenrohr (12) mit einem distalen (14) und einem proximalen Ende und mit einer ersten Elektrode (22) und wenigstens einer zweiten Elektrode (24, 26, 28a, 28b, 28c), wobei das Kanülenrohr (12) einen Kanülenrohrkörper (18) und eine die erste und die zweite Elektrode (22, 24) gegeneinander elektrisch isolierende Schicht (20) aufweist, wobei das distale Ende (12) des Kanülenrohrs (12) eine distale Spitze (16) aufweist, wobei die erste Elektrode (22) durch den Kanülenrohrkörper (18) gebildet ist und wobei die erste Elektrode (22) und die zweite Elektrode (24) mit einer Bioimpedanzmesseinheit verbindbar sind, wobei an dem proximalen Ende des Kanülenrohrs (12) ein Ansatz angeordnet ist, welcher einen elektrisch kontaktierenden Anschluss für die Elektroden (22, 24) aufweist und wobei die erste Elektrode (22) und die zweite Elektrode (24) von dem distalen Ende (14) bis zu dem Ansatz verlaufen,
**dadurch gekennzeichnet, dass** die erste Elektrode (22) und die zweite Elektrode (24) mittels eines Schalters wahlweise mit einer Stromversorgung oder mit der Bioimpedanzmesseinheit verbindbar sind.

2. Multipolare Kanüle (10, 10') nach Anspruch 1,
**dadurch gekennzeichnet, dass** die multipolare Kanüle eine Auswerteeinheit aufweist oder mit einer Auswerteeinheit verbunden ist, die ausgebildet ist, die von den Elektroden kommenden elektrischen Signale auszuwerten und ein Anzeigesignal, beispielsweise in Form eines akustischen oder optischen Anzeigesignals, zu erzeugen.

3. Multipolare Kanüle (10, 10') nach einem der vorhergehenden Ansprüche,
**dadurch gekennzeichnet, dass** die elektrisch isolierende Schicht (20) eine Dicke von wenigen Mikrometern, vorzugsweise eine Dicke von weniger als einem Mikrometer, aufweist.

4. Multipolare Kanüle (10, 10') nach einem der vorhergehenden Ansprüche,
**dadurch gekennzeichnet, dass** die zweite Elektrode (24, 28a, 28b, 28c) eine Dicke von wenigen Mikrometern, vorzugsweise eine Dicke von weniger als einem Mikrometer, aufweist.

5. Multipolare Kanüle (10, 10') nach einem der vorhergehenden Ansprüche,
**dadurch gekennzeichnet, dass** die elektrisch isolierende Schicht (20) aus Parylen ist.

## Claims

1. Multipolar cannula (10, 10'), having a cannula tube (12) with a distal (14) and a proximal end, and having a first electrode (22) and at least one second electrode (24, 26, 28a, 28b, 28c), wherein the cannula tube (12) comprises a cannula tube body (18) and a layer (20) which electrically isolates the first and the second electrode (22, 24) from each other, wherein the distal end (12) of the cannula tube (12) comprises a distal tip (16), wherein the first electrode (22) is formed by the cannula tube body (18) and wherein the first electrode (22) and the second electrode (24) are connectable by means of a bioimpedance measurement unit, wherein an attachment is arranged on the proximal end of the cannula tube (12) which comprises an electrically contacting connection for the electrodes (22, 24) and wherein the first electrode (22) and the second electrode (24) extend from the distal end (14) to the attachment,
**characterized in that** the first electrode (22) and the second electrode (24) are electively connectable to a power supply or to the bioimpedance measurement unit by means of a switch.

2. Multipolar cannula (10, 10') in accordance with claim 1,
**characterized in that** the Multipolar cannula comprises a processing unit or is connected to a processing unit which is implemented to process the electric signals coming from the electrodes and to generate an indication signal, for example, in the form of an acoustic or optical indication signal.

3. Multipolar cannula (10, 10') in accordance with either of the preceding claims,
**characterized in that** the electrically isolating layer (20) comprises a thickness of a few micrometers, preferably a thickness of less than one micrometer.

4. Multipolar cannula (10, 10') in accordance with any of the preceding claims, **characterized in that** the second electrode (24, 28a, 28b, 28c) comprises a thickness of a few micrometers, preferably a thickness of less than one micrometer.

5. Multipolar cannula (10, 10') in accordance with any of the preceding claims, **characterized in that** the electrically isolating layer (20) is made of parylene.

## Revendications

1. Canule multipolaire (10, 10') comportant un tube de canule (12) avec une extrémité distale (14) et une extrémité proximale ainsi qu'une première électrode (22) et au moins une seconde électrode (24, 26, 28a, 28b, 28c),
- le tube de canule (12) ayant un corps de tube de canule (18) et une couche électro-isolante (20) isolant la première et la seconde électrode (22, 24) l'une par rapport à l'autre,
- l'extrémité distale (14) du tube de canule (12) ayant une pointe distale (16),
- la première électrode (22) est formée par le corps du tube de canule (18), et
- la première électrode (22) et la seconde électrode (24) peuvent être reliées par une unité de mesure de bio-impédance,
- l'extrémité proximale du tube de canule (12) ayant un appendice qui comporte un branchement électrique des électrodes (22, 24), et
- la première électrode (22) et la seconde électrode (24) vont de l'extrémité distale (14) jusqu'à l'appendice,
canule **caractérisée en ce que**
la première électrode (22) et la seconde électrode (24) peuvent être reliées par un commutateur, soit à une alimentation électrique soit à une unité de mesure de bio-impédance.

2. Canule multipolaire (10, 10') selon la revendication 1,
**caractérisée en ce que**
la canule multipolaire a une unité d'exploitation ou est reliée à une unité d'exploitation conçue pour exploiter les signaux électriques venant des électrodes et générer un signal d'affichage, par exemple, sous la forme d'un signal d'affichage acoustique ou optique.

3. Canule multipolaire (10, 10') selon l'une des revendications précédentes,
**caractérisée en ce que**
la couche d'isolation électrique (20) a une épaisseur au moins de quelques microns, de préférence, une épaisseur inférieure à un micron.

4. Canule multipolaire (10, 10') selon l'une des revendications précédentes,
**caractérisée en ce que**
la seconde électrode (24, 28a, 28b, 28c) a une épaisseur au moins de microns, de préférence une épaisseur inférieure à un micron.

5. Canule multipolaire (10, 10') selon l'une des revendications précédentes,
**caractérisée en ce que**
la couche d'isolation électrique (20) est du parylène.
